# EUROPÄISCHE PATENTANMELDUNG

(11) **EP 0 639 554 A1**
(43) Veröffentlichungstag der Anmeldung: **22.02.1995**
(21) Anmeldenummer: 94117013.6
(22) Anmeldetag: 03.07.1992
(51) Int. Cl.: C07C 41/16, C07C 47/575, C07C 43/215, C07C 45/71

(54) **Verfahren zur Herstellung von Propargylethern**

(30) Priorität: 16.07.1991 DE 4123535
(62) Teilanmeldung aus: 92111299.1
(71) Anmelder: BAYER AG, 51368 Leverkusen (DE)
(72) Erfinder: Köhler, Burkhard, Dr., D-47829 Krefeld (DE); Dujardin, Ralf, Dr., D-47877 Willich (DE); Ebert, Wolfgang, Dr., D-47800 Krefeld (DE)

(57) **Zusammenfassung**

Die Erfindung betrifft ein Verfahren zur Herstellung von aromatischen Propargylethern durch Umsetzung von phenolischen Verbindungen mit 1 bis 2 Molen Propargylchlorid, bezogen auf 1 Mol phenolische OH-Gruppen, in Gegenwart von 1 bis 2 Molen Base, bezogen auf 1 Mol phenolische OH-Gruppen in organischen Lösungsmitteln und in Gegenwart eines Eisenkatalysators.

## Beschreibung

Die Erfindung betrifft ein Verfahren zur Herstellung von Propargylethern mit Eisenkatalyse.

Propargylether sind für die Herstellung von hydrophoben Duromeren von Interesse, die die Vorteile der Epoxyharze mit dem Vorteil geringer Wasseraufnahme verbinden. In High Performance Polymers 2(1) (1990), 67-77 wird diese Duromerklasse und die herkömmlichen Verfahren zur Herstellung der als Bausteine verwendeten Propargylether beschrieben. Einige der Verfahren führen zur Bildung der unerwünschten Claisen-Umlagerungsprodukte, andere Verfahren benötigten lange Reaktionszeiten.

Daher sind Verfahren von Interesse, die eine kurze Reaktionsdauer bei schonenden Bedingungen ermöglichen.

Ferner sind neue Propargylether mit hohem Cycloaliphatenanteil von Interesse, die die Herstellung von potentiell noch hydrophoberen Duromeren ermöglichen.

Überraschenderweise wurde nun gefunden, daß die Umesterung von Phenolen, Bisphenolen oder Polyphenolen mit Propargylhalogeniden durch Zusatz von Eisen oder Eisenverbindungen beschleunigt wird.

Gegenstand der Erfindung ist daher ein Verfahren zur Herstellung von aromatischen Propargylethern, in dem phenolische Verbindungen, vorzugsweise Mono-, Di-, der Polyphenole mit bezogen auf 1 Mol phenolische OH-Gruppen 1-2 Mole Propargylhalogenid in Gegenwart von bezogen auf 1 Mol phenolische OH-Gruppen 1-2 Mole Base
in Lösungsmitteln, vorzugsweise in aliphatischen C₁-C₄-Alkoholen, wie z.B. Isopropanol oder in Ketonen, wie z.B. Aceton, Methylethylketon, Cyclohexanon, oder in Nitrilen, wie z.B. Acetonitril, oder in Amiden, wie z.B. Dimethylformamid oder Dimethylacetamid, oder in Lactamen, wie z.B. N-Methylpyrrolidinon, N-Methylcaprolactam, oder in Harnstoffen, wie z.B. Tetramethylharnstoff, N,N'-Dimethylpropylenharnstoff, N,N'-Dimethylimidazolidinon oder in Dimethylsulfoxid,
umgesetzt werden, dadurch gekennzeichnet, daß der Reaktionslösung, bezogen auf 1 Mol phenolische OH-Gruppen 0,0001-0,01 Mol Eisen in elementarer Form oder in Form von Eisenverbindungen, vorzugsweise von EisenII- oder EisenIII-salzen, wie z.B. Oxiden, Sulfiden, Halogeniden, Nitraten, Sulfaten, Formiaten, Oxalaten, Tartraten, Acetaten, aliphatischen C₃-C₂₂-Carboxylaten, aromatischen C₆-C₁₄-Carboxylaten, besonders bevorzugt in Form einer im Lösungsmittel der Reaktion löslichen Eisenverbindungen, zugesetzt werden.

Beispiele für erfindungsgemäß umsetzbare Monophenole sind Phenole, Kresole, Xylenole, 2- oder 4-Phenylphenol, Octylphenole, Nonylphenole, Dodecylphenole, 2-Hydroxybenzaldehyd, 4-Hydroxybenzaldehyd, 2-, 3- oder 4-Hydroxybenzoesäure oder deren Ester oder Amide, 4-Hydroxyzimtsäure oder deren Ester oder Amide.

Beispiel für erfindungsgemäß umsetzbare Diphenole sind Bishydroxyphenylmethane (alle möglichen Isomeren), 2,2-Bis(4-hydroxyphenyl)propan, 2,2-Bis(4-hydroxyphenyl)hexafluorpropan, Hydrochinon, Resorcin, Brenzkatechin, 4,4'-Dihydroxybiphenyl, 2,2'-Dihydroxybiphenyl, Dihydroxyterphenyl, Dihydroxyquaterphenyl, 4,4'- oder 3,3'-Dihydroxydiphenylether, 4,4'-Dihydroxydiphenylsulfon, 4,4'-Dihydroxydiphenylsulfid, 3,4- oder 2,4-Dihydroxybenzaldehyd, 2,4- oder 2,4'- oder 4,4'-Dihydroxybenzophenon, 2,4- oder 3,4-Dihydroacetophenon, 4,4'-Dihydroxystilben, 1,2-Bis-(4-hydroxyphenol)ethan, 1,2-Bis-(4-hydroxyphenyl)-propan.

Beispiele für erfindungsgemäß umsetzbare Diphenole, wobei die Umsetzung zu den erfindungsgemäßen Propargylethern mit hohem Anteil an cycloaliphatischen Gruppen führt, sind Bis(4-hydroxyphenyl)cyclohexan, 1,1-Bis(4-hydroxyphenyl)-3,3,5-trimethylcyclohexan, Kondensationsprodukte aus Phenol und Dicyclopentadien, Kondensationsprodukte aus Phenol und Terpene, wie z.B. Limonen, besonders bevorzugt 1,1-Bis(4-hydroxyphenyl)-3,3,5-trimethylcyclohexan.

Beispiele für erfindungsgemäß umsetzbare Polyphenole sind Kondensationsprodukte aus Fomaldehyd und Phenolen, wie z.B. Novolake.

Beispiele für erfindungsgemäß verwendete Base sind Alkalialkoholate, Alkalicarbonate, Alkalihydroxide, tertiäre Amine oder basische Ionenaustauscher.

Beispiele für erfindungsgemäß verwendete Lösungsmittel sind Methanol, Ethanol, n-Propylalkohol, Isopropylalkohol, Butanole, Aceton Cyclohexanon, Acetonitril.

Das erfindungsgemäße Verfahren kann innerhalb von 0,1 bis 10 Stunden, vorzugsweise 0,5 bis 5 Stunden, bei Temperaturen von 0°C bis 100°C, vorzugsweise von 20°C-80°C, und bei einem Druck von 0,5 bis 10 bar, vorzugsweise bei Normaldruck, durchgeführt werden.

Es zeichnet sich durch eine hohe Reaktionsgeschwindigkeit aus. Ferner treten unerwünschte Umlagerungen nur in geringem Ausmaß auf.

### Beispiele

### Vergleichsbeispiel 1

### Herstellung von 4-Propargyloxybenzaldehyd

Man mischt 122 g 4-Hydroxybenzaldehyd, 1000 ml Aceton, 138 g Kaliumcarbonat und 150 g Propargylbromid. Man erhitzt 4 Stunden auf 60°C, filtriert ausgefallene Bestandteile ab, engt ein, nimmt in Methylenchlorid auf, schüttelt mit 10 proz. NaOH aus und engt die organische Phase ein. Man erhält 20 bis 35 g 4-Propargyloxybenzaldehyd.

### Beispiel 1 (erfindungsgemäßes Verfahren)

### Herstellung von 4-Propargyloxybenzaldehyd

Man verfährt wie in Vergleichsbeispiel 1 beschrieben, setzt aber der Mischung 0,05 g Eisen(III)chlorid zu. Man erhält 109 g 4-Propargyloxybenzaldehyd in NMR-reiner Form.

### Beispiel 2 (erfindungsgemäßes Verfahrens)

### Herstellung von 2,2-Bis(4-Propargyloxyphenyl)propan

Man mischt 228 g Bisphenol-A, 300 ml Isopropanol, 360 g einer 30 proz. Lösung von Natriummethanolat in Methanol, 0,05 g Eisen(III)chlorid und 200 g Propargylchlorid. Es kommt zu einer exothermen Reaktion, nach deren Dauer von ca. 15 min. die Hauptmenge des Produktes entstanden ist (DC-Kontrolle). Man läßt 4 Stunden nachreagieren, filtriert ab und engt ein. Man erhält 275 g des Produktes (durch NMR-Spektroskopie charakterisiert).

### Beispiel 4 (Propargylether mit hohem Cycloaliphatenanteil)

### Herstellung von 1,1-Bis(4-propargyloxyphenyl)-3,3,5-trimethylcyclohexan

Man verfährt wie in Beispiel 2 beschrieben, wobei das Bisphenol-A durch 319 g 1,1-Bis(4-hydroxyphenyl)3,3,5-trimethylcyclohexan ersetzt wird. Man erhält 326 g Produkt (durch NMR-Spektroskopie charakterisiert).

## Patentansprüche

1. Verfahren zur Herstellung von aromatischen Propargylethern durch Umsetzung von phenolischen Verbindungen mit 1 bis 2 Molen Propargylchlorid, bezogen auf 1 Mol phenolische OH-Gruppen, in Gegenwart von 1 bis 2 Molen Base, bezogen auf 1 Mol phenolische OH-Gruppen in organischen Lösungsmitteln, dadurch gekennzeichnet, daß die Umsetzung in Gegenwart von Eisenkatalysator durchgeführt wird.

2. Verfahren nach Anspruch 1, dadurch gekennzeichnet, daß als phenolische Verbindungen Mono-, Di- oder Polyphenole eingesetzt werden.

3. Verfahren nach Anspruch 1, dadurch gekennzeichnet, daß als organische Lösungsmittel C₁-C₄-Alkohole, Ketone, Nitrile, Amide, Lactame oder Harnstoffe eingesetzt werden.

4. Verfahren nach Anspruch 1, dadurch gekennzeichnet, daß als Eisenkatalysator von 0,0001 bis 0,1 Mol Eisen, bezogen auf 1 Mol phenolische OH-Gruppen in elementarer Form oder in Form von Eisenverbindungen eingesetzt wird.

5. Verfahren nach Anspruch 4, dadurch gekennzeichnet, daß als Eisenkatalysator Eisen II- oder Eisen III-Salze eingesetzt werden.

6. Verfahren nach Anspruch 1, dadurch gekennzeichnet, daß die Umsetzung bei 0 bis 100°C durchgeführt wird.
